# EUROPEAN PATENT APPLICATION

(11) **EP 0 942 230 A1**
(43) Date of publication of application: **15.09.1999**
(21) Application number: 98108676.2
(22) Date of filing: 13.05.1998
(51) Int. Cl.: F22B 1/28, A47L 11/04

(54) **Multifunotional apparatus for delivering two different types of steam**

(30) Priority: 13.03.1998 IT MI980521
(71) Applicant: C.D.S. Cleaning & Disinfection Systems S.r.l., 20131 Milano (IT)
(72) Inventor: Zaniboni, A., S.D.S. Clean. & Disin. Sys. S.R.L., 20131 Milano (IT)
(74) Representative: Cicogna, Franco

(57) **Abstract**

The present invention relates to a multifunctional apparatus for delivering two different types of steam, one at a lower temperature for cleaning and the other at a greater temperature for disinfecting surfaces and the like, which comprises a steaming boiler coupled to a water inlet duct, and provided with heating means.

At the outlet of the boiler a switching device (11) is provided for selectively communicating the boiler with a first duct (12) for delivering the steam at a lower temperature and with a duct (20) for supplying a overheater (21) coupled to a second delivery duct (30) for delivering the steam at a greater temperature.

## Description

### BACKGROUND OF THE INVENTION

The present invention relates to a multi-functional apparatus for delivering two different types of steam, one at a lower temperature for cleaning and the other at a greater temperature for disinfecting surfaces and the like.

Prior steam generating apparatus for generating steam for cleaning surfaces and the like, conventionally comprise a box-like body inside of which a water tank is arranged.

The water tank is coupled, through a pump, to a steaming boiler.

The steaming boiler is coupled, at the outlet thereof, to a duct, in turn coupled to a jet delivery device, conventionally formed by a delivery gun or lance, ejecting the steam jet onto the surface to be cleaned.

The mentioned prior apparatus is usually designed to deliver steam at about 100°C.

This temperature, while providing a proper cleaning of the surfaces, does not allow to perfectly disinfect said surfaces, since, as is known, several bacteria can resist up to temperatures of 150-160°C.

Moreover, by using steam having a temperature of 100°C, said steam may frequently condense on the surfaces being cleaned, thereby providing a not fully satisfactory cleaning and generating dirty stains along the processed surface.

Thus, it has been actually found that, in order to carry out a proper cleaning, it would be necessary to use steam having at least a temperature of 130-140°C.

This temperature would allow fats and incrustations possibly present on the surface being processed to be easily dissolved.

For the so-called "open sky" disinfection, it has been found that it would be absolutely necessary to process the surface to be cleaned by steam having a temperature greater than 200°C.

Microbiologic measurement tests have demonstrated that such a temperature is effectively suitable to kill all of the microorganisms, thereby providing a microorganism level constantly corresponding to zero.

### SUMMARY OF THE INVENTION

Accordingly, the aim of the present invention is to overcome the above mentioned problems, by providing a multifunctional apparatus for delivering two different types of steam, allowing to perfectly clean surfaces as well as to fully disinfect the surfaces being processed.

Within the scope of the above mentioned aim, a main object of the present invention is to provide such an apparatus able to deliver steam at a temperature of both 140-150°C and 220-250°C, thereby safely removing dirty as well as killing even very resistant bacteria.

Another object of the present invention is to provide such a multifunctional steam delivery apparatus which, owing to its constructional features, is very reliable and safe in operation.

Yet another object of the present invention is to provide such a multifunctional steam delivery apparatus which can be easily used both in a home environment and for cleaning hospitals, restaurant kitchens, supermarkets and the like.

According to one aspect of the present invention, the above mentioned aim and objects, as well as yet other objects, which will become more apparent hereinafter, are achieved by a multi-functional apparatus for delivering two different types of steam, one at a lower temperature for cleaning and one at a greater temperature for disinfecting surfaces and the like, characterized in that said apparatus comprises a steaming boiler, coupled to a water inlet duct and provided with first heating means.

At the outlet of the boiler a switching device is provided, for selectively communicating said boiler with a first delivery duct for delivering steam at a lower temperature and a supplying duct for supplying an overheating device, coupled to a second steam delivery duct for delivering steam at a greater temperature.

### BRIEF DESCRIPTION OF THE DRAWINGS

Further characteristics and advantages of the present invention will become more apparent hereinafter from the following detailed disclosure of a preferred, though not exclusive, embodiment of a multifunctional apparatus for delivering two different types of steam, which is illustrated, by way of an indicative, but not limitative, example, in the accompanying drawings, where:

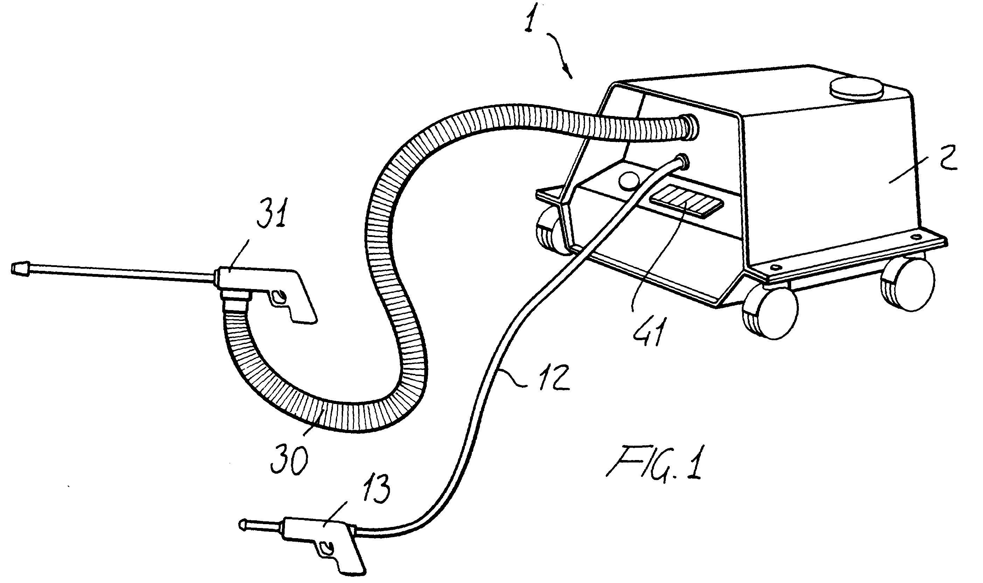

Figure 1 is a schematic perspective view illustrating the apparatus according to the present invention; and

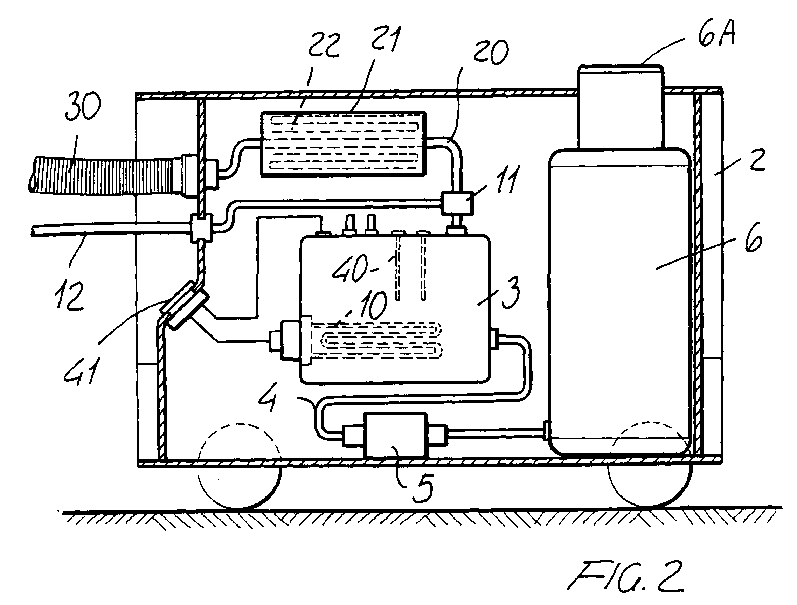

Figure 2 is a cross-sectional view illustrating the apparatus and its main components.

### DESCRIPTION OF THE PREFERRED EMBODIMENT

With reference to the number references of the above mentioned figures, the multifunctional apparatus for delivering two different types of steam, according to the present invention, which has been generally indicated by the reference number 1, comprises a wheeled body 2, inside which a steaming boiler 3 is provided, said steaming boiler 3 being coupled to a water inlet duct 4, including a pump 5 and communicating with a water tank 6.

Inside the steaming boiler 3 first heating means comprising a first electric resistance 10 operating for increasing the temperature in said boiler are provided.

At the outlet of the boiler, a switching device 11 is provided for selectively communicating said boiler with a first steam delivery duct 12 for delivering steam at a lower temperature and, more specifically, at a boiler outlet temperature of 150-160°C, which temperature will decrease to 130-140°C at the gun or lance delivery element, indicated by the reference number 13.

The switching device 11, advantageously comprising a solenoid valve, will allow to supply steam at the above mentioned temperature to the first duct including a thermally insulated pipe, sending the steam to the first steam spraying gun or lance 13.

The switching device 11, as shown, is coupled to a supply duct 20 for supplying a overheater 21, including second heating means 22, designed for increasing the steam temperature at the outlet of the boiler, to a temperature value of 220-200°C.

In order to enhance the thermal exchange, the path followed by the steam in said overheater, is not a merely linear type of path, but it can be designed as a multiple branch pipe, or a spiral-like path, to provide a further enhanced thermal exchange.

At the outlet of said overheater a second steam delivery duct, indicated by the reference number 30 and coupled to a second steam delivery gun 31 is provided.

Whereas the first lower temperature steam duct can comprise a spiral or coil pipe made of a simple synthetic rubber material, adapted to resist against a temperature of 150-160°C, the second greater temperature steam delivery duct is advantageously made with a suitable inner insulating material, in order to properly resist against and hold the desired temperature levels.

A like insulating material will be applied to the second steam delivery gun or lance, in order to prevent the user from burning.

Advantageously, the overheater 21 is provided with a thermally conductive material block, for example made of aluminium, affected by the second heating means 22, thereby providing an optimum thermal level.

In this connection it should be moreover be pointed out that said overheater 21 can be arranged both within and outside of the body of the apparatus, at any desired position thereon.

Inside the boiler 3 a water level sensor, indicated by the reference number 40, which can be advantageously calibrated both for a regular water and for distilled water is provided.

To use distilled water, which use is permitted by the specifically designed sensor herein used, is very important, since it will allow to reduce to a minimum possible accumulations of limestone, which would be inevitable as water is caused to boil in a closed vessel which can not be frequently washed.

The wheeled body 2 is provided at the top thereof with an opening therefrom the top end portion of the tank 6 projects, said tank being advantageously provided with a closure plug 6a which can be easily removed.

On the front of the wheeled body, a control panel indicated by the reference number 41 is provided, said control panel including thereon a plurality of push-buttons for operating the apparatus, as well as several pilot lamps and control devices as well as the operating element for the switching device 11, to supply steam toward either the first or second steam delivery duct.

Moreover, inside the steaming boiler a temperature sensor as well as a pressure sensor for controlling said boiler can be provided.

The operation of the apparatus according to the invention is very simple, since as the first and second heating means 10 and 22 are power supplied, then the water introduced from the tank 6, through the pump 5, into the steaming boiler will be quickly heated so as to generate steam: said steam will exit the boiler at a temperature of 150-160°C, thereby it can be directly supplied to the first steam delivery duct 12.

In the case in which an overheated steam is desired, then the switching device will be adjusted so as to cause the generated steam to be supplied to the overheater 21, for increasing the steam temperature at a temperature value from 220°C to 250°C.

Then, the steam jet impinging on the surface being cleaned, will assure both a proper surface cleaning and a full disinfection thereof, since at the mentioned temperature of 220-250°C any resistant bacteria present on said surface will be safely eliminated.

From the above disclosure it should be apparent that the invention fully achieves the intended aim and objects.

In particular, the subject apparatus is particularly suitable for use in hospitals, restaurant kitchens and the like, supermarkets, hygienic sanitary systems, and in all the cases in which it is requires to fully clean surfaces as well as to disinfect and sterilize said surfaces.

The invention, as disclosed, is susceptible to several modifications and variations, all of which will come within the scope of the invention.

Moreover, all the constructional details can be replaced by other technically equivalent elements.

In practicing the invention, the used materials, provided that they are compatible to the intended application, as well as the contingent size and shapes, can be any, according to requirements.

## Claims

1. A multifunctional apparatus for delivering two different types of steam, characterized in that said apparatus comprises a steaming boiler, coupled to a water inlet duct and provided with first heating means, at the outlet of said steaming boiler being provided a switching device for selectively communicating said steaming boiler with a first lower temperature steam delivery duct, and with a steam supplying duct of an overheater, coupled to a second greater temperature steam delivery duct.

2. A multifunctional apparatus, according to the preceding claim, characterized in that said lower temperature is from 130°C to 160°C.

3. A multifunctional apparatus, according to the preceding claims, characterized in that said greater temperature is from 220°C to 250°C.

4. A multifunctional apparatus, according to one or more of the preceding claims, characterized in that said apparatus comprises a water tank coupled to said steaming boiler through a pump.

5. A multifunctional apparatus, according to one or more of the preceding claims, characterized in that said apparatus comprises a solenoid valve for controlling said switching device.

6. A multifunctional apparatus, according to one or more of the preceding claims, characterized in that said first heating means comprise an electric resistance, arranged inside said steaming boiler.

7. A multifunctional apparatus, according to one or more of the preceding claims, characterized in that said overheater comprises second heating means including coiled electric resistances, embedded in a thermally conductive material block.

8. A multifunctional apparatus, according to one or more of the preceding claims, characterized in that said apparatus comprises first and second steam delivery means coupled to a first and second steam delivery duct.

9. A multifunctional apparatus, according to one or more of the preceding claims, characterized in that said apparatus comprises sensing means for sensing the water level in said steaming boiler, said sensing means being adapted to be calibrated for either regular water or distilled water use.

10. A multifunctional apparatus, according to one or more of the preceding claims, characterized in that said apparatus comprises sensing means for sensing the temperature inside said steaming boiler.

11. A multifunctional apparatus, according to one or more of the preceding claims, characterized in that said apparatus comprises steam pressure sensing means inside said steaming boiler.

12. A multifunctional apparatus, according to one or more of the preceding claims, characterized in that said first lower temperature steam delivery duct comprises a synthetic rubber material coiled pipe.

13. A multifunctional apparatus, according to one or more of the preceding claims, characterized in that said second steam delivery duct is provided with an inner thermally insulating material, in order to resist against and hold temperatures from 220°C to 250°C.

14. A multifunctional apparatus, according to one or more of the preceding claims, characterized in that said apparatus comprises delivery means arranged at the ends of said first and second steam delivery ducts.

15. A multifunctional apparatus, according to one or more of the preceding claims, characterized in that said apparatus comprises an overheater, which can be arranged either in the body of said apparatus or outside of said body at any desired position thereon.

16. A multifunctional apparatus for delivering two different types of steam, characterized in that said apparatus comprises specifically designed constructional elements substantially as broadly disclosed and illustrated and for the intended aim and objects.
